# EUROPÄISCHE PATENTSCHRIFT

(11) **EP 0 677 299 B1**
(45) Veröffentlichungstag und Bekanntmachung des Hinweises auf die Patenterteilung: **04.07.2001**
(21) Anmeldenummer: 95105429.5
(22) Anmeldetag: 11.04.1995
(51) Int. Cl.: A61M 25/00

(54) **Verpackung eines medizinischen Instruments**
Package for a medical instrument
Emballage pour instrument médical

(30) Priorität: 13.04.1994 DE 4412754
(43) Veröffentlichungstag der Anmeldung: 18.10.1995
(73) Patentinhaber: VIA LOG Medikalprodukte GmbH Kosmetik - Medien, D-75378 Bad Liebenzell (DE)
(72) Erfinder: Hutzler, Martin, D-75378 Bad Liebenzell (DE)
(74) Vertreter: KOHLER SCHMID + PARTNER

(56) Entgegenhaltungen:
- FR-A- 2 386 312
- GB-A- 2 033 231
- US-A- 3 967 728

## Beschreibung

Die Erfindung bezieht sich auf die Verpackung eines mit einem Benetzungsmittel behandelbaren medizinischen Instruments, insbesondere eines Katheters zum Einführen in die Urethra, wobei das medizinische Instrument mindestens von einer Verpackung steril umhüllt ist und innerhalb der Verpackung eine tunnelartige Öffnung zum Herausführen des medizinischen Instruments vorgesehen ist .

Diese tunnelartige Öffnung ist so ausgebildet, daß das medizinische Instrument seitliche geführt über das Lumen der Öffnung heraussohiebbar ist. Die Verpackung selbst weist mindestens an einer der beiden Querseiten Öffnungshilfen auf.

Eine derartige Verpackung ist durch die GB 2033231 A bekanntgeworden.

Derartige Verpackungen sind vielfach bekannt, weil medizinische Instrumente steril verpackt ihrem Anwendungsbereich zugeführt werden müssen. Die Verpackungen werden dann zerstört, wenn das medizinische Instrument benötigt wird. Die Verpackung gewährleistet, daß das in der Verpackung gelagerte Instrument bis zum Einsatz steril bleibt.

Handelt es sich bei dem medizinischen Instrument um einen Katheter, so ist es üblich, daß der Katheter bei Gebrauch aus der Verpackung herausgeschoben wird, indem man die Verpackung an einem Ende öffnet.

Beim Katheterismus ist es zur Reduzierung der Gleitreibung bekannt, daß man die Außenflächen des Katheters mit Gleitmittel oder einer Gleithilfe einstreicht, damit der Katheter möglichst schmerzfrei und ohne weitere Irritationen hervorzurufen in Hohlorgane des menschlichen Körpers eingeführt werden kann. Es ist auch bekannt, die Außenfläche eines medizinischen Instruments mit medizinischen Wirkstoffen zu benetzen, damit dann diese medizinischen Wirkstoffe im Hohlorgan an das an das Instrument angrenzende Gewebe abgegeben werden.

Soll das Einstreichen oder das Benetzen der Außenoberfläche eines Katheters möglichst gleichmäßig und mit einer kleinen Menge Gleithilfe erfolgen und möglich sein, so kann beispielsweise eine Benetzungsvorrichtung für medizinische Instrumente verwendet werden, wie sie aus der DE 41 41 787 C2 bekannt ist.

Bei dieser bekannten Benetzungsvorrichtung wird der Katheter durch eine Hülse geführt, die in das Lumen ragende Vorsprünge aufweist. Die Vorsprünge sind auf der Innenoberfläche gleichmäßig über den gesamten Umfang gesehen verteilt, sodaß das Gleitmittel oder die Gleithilfe in den Zwischenräumen der voneinander beabstandeten Vorsprünge speicherbar ist, und es ist gewährleistet, daß beim Hindurchführen eines Katheters durch die bekannte Benetzungsvorrichtung die gesamte Außenoberfläche des Katheters gleichmäßig und vollkommen benetzt wird. Damit werden die bekannten Irritationen beim Einführen eines Katheters in ein Hohlorgan weitgehend reduziert bzw. vollkommen aufgehoben.

Soll die bekannte Benetzungsvorrichtung, eine Hülse mit besonderen Merkmalen, verwendet werden, so muß sie entweder steril getrennt vom Katheter geschützt und gelagert werden oder sie muß in die Verpackung eingearbeitet werden. Diese Lagerung oder Anordnung in der Verpackung ist aufwendig und deshalb mit bestimmten Mindestkosten verbunden. Obwohl über diese Art der Benetzung stets gewährleistet ist, daß das medizinische Instrument vollkommen mit Gleithilfe in einer gleichmäßigen Dicke beschichtet ist, scheint eine derartige Benetzung zu kostenintensiv zu sein.

Nach der Lehre der GB 2033231 A weist die Verpackung eine trichterförmige Kammer auf, in die ein Benetzungsmittel nach dem Aufreißen der Verpackung eingebracht werden kann. Das Benetzungsmittel wird undefiniert in der Kammer deponiert. Die Kammer ist durchgängig mit einer kanalartigen Verengung verbunden, in der sich ein herausschiebbares medizinisches Instrument befindet. Wenn das medizinische Instrument aus der Verengung herausgeführt wird, taucht das medizinische Instrument innerhalb der Kammer in das Benetzungsmittel ein. Durch das Hindurchführen des medizinischen Instruments durch die Kammer hindurch aus der Verpackung heraus ist nicht gewährleistet, daß die Außenfläche des medizinischen Instruments gleichmäßig mit dem Benetzungsmittel kontaktiert wird.

Aufgabe der vorliegenden Erfindung ist es deshalb, eine gleichmäßigere Benetzung des medizinischen Instruments beim Herausführen aus der Verpackung zu erreichen.

Gelöst wird diese Aufgabe dadurch, daß die Öffnungshilfen zum Freilegen der tunnelartigen Öffnung vorgesehen sind, daß das Benetzungsmittel im freigelegten Zustand der tunnelartigen Öffnung unmittelbar in die nicht vom Katheder besetzte tunnelartige Öffnung einbringbar ist, und daß die tunnelartige Öffnung so ausgebildet ist, daß nach Einbringen des Benetzungsmittels dieses im Bereich der tunnelartigen Öffnung gespeichert wird.

Die erfindungsgemäße Verpackung hat damit den wesentlichen Vorteil, daß der zum Schutz des Katheters sowieso benötigten Verpackung eine weitere Funktion zugeordnet wird:
- Die Verpackung wird im Bereich der Öffnung so ausgebildet, daß bei offener Öffnung entweder Gleitmittel oder eine Gleithilfe von außen in die Öffnung eingespritzt wird oder
- der offenen Öffnung wird von dem Innenraum der Verpackung die Gleithilfe zugeführt.

Die Öffnung muß so beschaffen sein, daß sie in ihrem Lumen eine gewisse Menge Gleithilfe so speichern kann, daß diese Gleithilfe an die Außenoberfläche eines durch die Öffnung geführten Katheters abgebbar ist.

Mit den Öffnungshilfen läßt sich beispielsweise einenends die Verpackung gezielt öffnen, indem die Verpackungsstreifen an ihren verschweißten Rändern wieder voneinander gelöst werden. An den dann zugänglichen Kathetertrichter läßt sich beispielsweise ein Urinbeutel anschließen. Mit der anderenends ausgebildeten Öffnungshilfe läßt sich die Verpackung beispielsweise so weit öffnen, daß die Öffnung der Verpakkung freigelegt wird. In diese Öffnung kann dann die Gleithilfe eingebracht werden. In die Verpackung kann auch eine Ampulle, die die Gleithilfe enthält, eingeschweißt sein. Die ebenfalls sterile Ampulle wird dann zuerst aus der Verpakkung genommen, und danach wird die Gleithilfe in die offengelegte Öffnung eingespritzt.

Über Öffnungshilfen mit geführten Aufreißlinien läßt sich die erfindungsgemäße Verpackung an beliebigen Stellen über vordefinierte Öffnungswege öffnen.

Dabei ist vorteilhaft, daß die Verpackung in der Größe an das medizinische Instrument angepaßt ist und daß die Verpakkung mindestens auf einer Seite weitgehend durchsichtig ausgebildet ist. Dies hat den Vorteil, daß man die Lage des Katheters in der Verpackung stets sehen kann, und auch die Lage des an sich bekannten Katheters zur Öffnung der Verpakkung ist stets einsehbar. Auch kann so überprüft werden, wieviel Gleitmittel im Öffnungsbereich ist.

In einer vorteilhaften Ausgestaltung der Verpackung ist die Verpackung aus einem ersten und einem zweiten Verpackungsstreifen gebildet, die aufeinanderliegend im Randbereich allseitig lösbar miteinander verbunden sind. Bei den Verpakkungsstreifen handelt es sich um die Packstoffe, aus denen die Verpackung hergestellt wird.

Über eine derartige Ausbildung der Verpackung ist es mit einfachsten Mitteln möglich, besonders effektive Öffnungsquerschnitte zu schaffen, die Gleithilfe in geringster aber ausreichender Menge an den Katheter abgeben. Die Verpakkungsstreifen werden derart an ihren Rändern miteinander verschweißt, daß innerhalb der Verpackungsstreifen ein Raum entsteht, in dem der Katheter steril gelagert werden kann. Die Verpackungsstreifen sind im Öffnungsbereich gezielt über einfachste kostengünstige Mittel so miteinander verbindbar, daß eine Art rohrförmige Verbindung als Öffnung für den hindurchzuführenden Katheter entsteht. In diesen Rohrabschnitt kann die Gleithilfe eingebracht werden.

Besonders vorteilhaft ist es, wenn die Verpackungsstreifen derart im Bereich der Öffnung zusammengefügt sind, daß die seitliche Führung des medizinischen Instruments gewährt ist. Damit ist gewährleistet, daß das in der Öffnung befindliche Gleitmittel bzw. die Gleithilfe an der Außenoberfläche des Katheters zur Anlage kommen kann.

In weiterer Ausgestaltung der Erfindung ist das Benetzungsmittel eine Gleithilfe oder ein einen medizinischen Wirkstoff beinhaltendes Gleitmittel, wobei diese Gleithilfe bzw. das Gleitmittel in einer Ampulle bzw. einer Kapsel gespeichert werden kann.

Eine Ampulle bzw. eine Kapsel kann mit einfachen Mitteln zwischen den Verpackungsstreifen innerhalb der Verpackung steril gelagert werden. Werden dem Gleitmittel noch medizinische Wirkstoffe hinzugefügt, so kann der Einsatzbereich der erfindungsgemäßen Verpackung noch erweitert werden.

In einer weiteren vorteilhaften Ausgestaltung der Erfindung ist unmittelbar hinter der Öffnung innerhalb der Verpackung eine unter Druck zerstörbare Gleithilfekapsel lagestabil angeordnet.

Dies hat den Vorteil, daß in die Öffnung selbst von außen kein Gleitmittel bzw. eine Gleithilfe eingespritzt werden muß, bevor der Katheter durch die Öffnung hindurchgeführt wird. Die Kapsel mit der notwendigen Menge Gleithilfe ist in die Verpackung, d. h. zwischen die Verpackungsstreifen lagestabil eingeschweißt, und soll ein Katheter durch die Öffnung hindurchgeführt werden, so wird mittels Fingerdruck die Gleithilfekapsel gesprengt und der Katheter kann durch die Gleithilfe hindurchgeführt und aus der Öffnung heraus ins Freie geschoben werden.

In weiterer Ausgestaltung der Erfindung ragen an der Verpakkung im Bereich der Öffnung über eine axiale Erstreckung Vorsprünge in das Lumen der Öffnung.

Dies hat den Vorteil, daß mit geringstem Aufwand ein ähnlicher Erfolg wie mit der Benetzungsvorrichtung nach DE 41 41 787 C2 erzielt werden kann. Zwar können über Rilleneinprägungen in die Verpackungsstreifen nicht derart reproduzierbare Vorsprünge wie bei der bekannten Benetzungsvorrichtung geschaffen werden, doch es bilden sich auch bei der erfindungsgemäßen Verpackung im Bereich der Öffnung Freiräume zwischen den Vorsprüngen aus, in die die Gleithilfe eindringen kann und dort speicherbar ist. Aus diesen Freiräumen läßt sich dann die Gleithilfe in definierten Mengen an die Außenoberfläche des durch die Öffnung hindurchgeführten Katheders abgegeben.

Weitere Vorteile ergeben sich aus der Beschreibung und der beigefügten Zeichnung.

Die erwähnten Ausführungsformen sind nicht als abschließende Aufzählung zu verstehen, sondern haben vielmehr beispielhaften Charakter.

Die Erfindung ist in der Zeichnung dargestellt und wird anhand von Ausführungsbeispielen näher erläutert. Es zeigt:
- Fig. 1: eine erfindungsgemäße Verpackung mit einem darin eingeschlossenen Katheter;
- Fig. 2: eine vergrößerte Darstellung der erfindungsgemäßen Verpackung der Fig. 1 im Bereich der Verpakkung mit der Öffnung, durch die der Katheter hindurchführbar ist;
- Fig. 3a: eine Ampulle, in der die Gleithilfe gespeichert ist, die in die Öffnung der erfindungsgemäßen Packung einspritzbar ist;
- Fig. 3b: eine Teilansicht einer erfindungsgemäßen Verpakkung nach Fig. 1 im Berreich der Öffnung mit einer Ampulle bzw. alternativ einer Kapsel, in der die Gleithilfe gespeichert ist;
- Fig. 4: einen Abschnitt einer erfindungsgemäßen Verpakkung mit einer Öffnung, die in das Lumen gerichtete Vorsprünge aufweist;
- Fig. 5: einen Abschnitt einer erfindungsgemäßen Verpakkung mit einer hinter der Öffnung innerhalb der Verpackung eingeschlossenen Gleithilfekapsel.

Die Figuren der Zeichnung zeigen den erfindungsgemäßen Gegenstand stark schematisiert und sind nicht maßstäblich zu verstehen. Die einzelnen Bereiche der Verpackung sind so dargestellt, daß ihr Aufbau gut gezeigt werden kann.

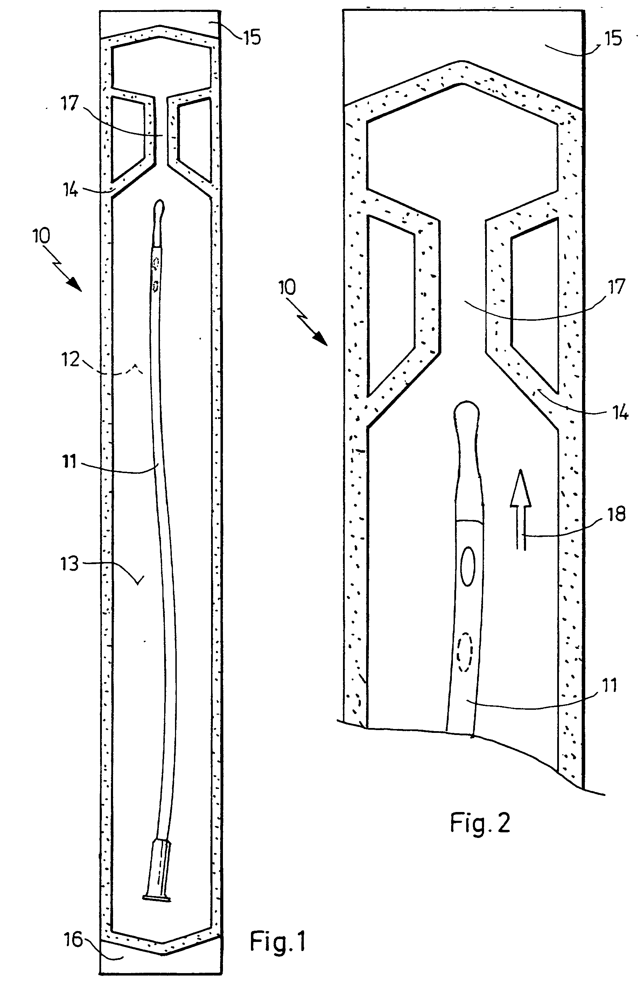

**Fig. 1** zeigt mit 10 eine Verpackung, in der ein Katheter 11 steril verpackt eingeschlossen ist. Der Katheter 11 ist ein an sich bekannter Katheter. Der Katheter 11 ist innerhalb der Verpackung 10 gehalten, die sich aus einem ersten Verpackungsstreifen 12 und einem zweiten Verpackungsstreifen 13 zusammensetzt. Die Verpackungsstreifen 12,13 sind in den punktierten Streifenbereichen 14 zusammengefügt, bevorzugt geklebt bzw. verschweißt. Die Verpackungsbereiche innerhalb der punktierten Streifenbereiche 14 bilden Hohlräume. Innerhalb der Verpackung 10 ist der Katheter 11 angeordnet.

Die Verpackung 10 weist am jeweiligen freien Ende eine erste Öffnungshilfe 15 und eine zweite Öffnungshilfe 16 auf. Über die Öffnungshilfen 15,16 kann die Verpackung 10 geöffnet werden.

Werden die Verpackungsstreifen 12, 13 im Bereich der ersten Öffnungshilfe 15 auseinandergezogen, so kann die Verpackung 10 zuerst bis zu einer Öffnung 17 geöffnet werden. Durch die Öffnung 17 kann der Katheter 11 hindurchgeschoben werden.

Wird über die zweite Öffnungshilfe 16 die Verpackung 10 geöffnet, so kann am anderen Ende des Katheters 11 beispielsweise ein Urinbeutel angeschlossen werden. Im hinteren Teil der Verpackung 10, im Bereich der zweiten Öffnungshilfe 16, kann auch weiteres Zubehör für den Katheter 11 steril verpackt sein.

Ist der Katheter 11 erfindungsgemäß benetzt, so können die Verpackungsstreifen 12, 13 vollkommen voneinander getrennt werden.

Bei den Verpackungsstreifen 12,13 ist zumindest ein Verpakkungsstreifen 12 oder 13 teilweise durchsichtig ausgebildet, damit die Lage des Katheters 11 innerhalb der Verpackung 10 und die Lage des Katheters 11 zur Öffnung 17 erkannt werden kann.

**Fig. 2** zeigt einen Teilabschnitt der Verpackung 10 aus Fig. 1. In der Fig. ist ein Ende der Verpackung 10 mit der ersten Öffnungshilfe 15 gezeigt. Die Verpackung 10 ist in den punktierten Streifenbereichen 14 zusammengehalten, und durch die punktierten Streifenbereiche 14 ist auch die Öffnung 17 innerhalb der Verpackung 10 definiert. Der Katheter 11 kann in Pfeilrichtung 18 durch die Öffnung 17 geschoben werden. Im gezeigten Zustand ist die Verpackung 10 geschlossen. Im Bereich der ersten Öffnungshilfe 15 sind die Verpackungsstreifen nicht aneinandergeklebt, sodaß Haltebereiche zum Öffnen der Packung 10 entstehen.

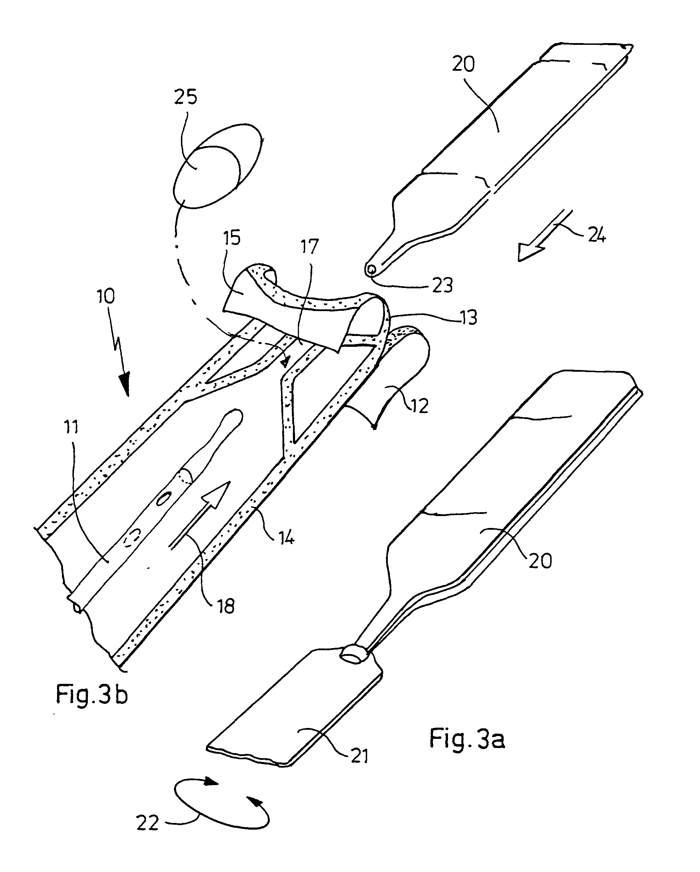

**Fig.3a** zeigt eine Ampulle 20, in der die Gleithilfe bzw. das Gleitmittel für die Benetzung der Außenoberfläche des Katheters gespeichert ist. Die Ampulle 20 ist bevorzugt aus Kunststoffmaterial, das elastische Eigenschaften aufweist. Die Ampulle 20 ist über eine Verschlußplatte 21 verschlossen. Wird die Verschlußplatte 21 in Pfeilrichtungen 22 verdreht, so bricht die Verschlußplatte 21 vom restlichen Teil der Ampulle 20 ab, und es entsteht eine Öffnung an der Ampulle 20, aus der die in der Ampulle 20 gespeicherte Gleithilfe herausgedrückt werden kann.

**Fig. 3b** zeigt die Zuordnung der Ampulle 20 zur Verpackung 10 bei geöffneter erster Öffnungshilfe 15.

Die Spitze der Ampulle 20 wird in die Öffnung 17 der Verpakkung 10 gedrückt und Gleithilfe 23 kann in die Öffnung 17 hineingepreßt werden. In der Fig. sind die ersten und zweiten Verpackungsstreifen 12,13 gut erkennbar, und sie sind bis zur Öffnung 17 auseinandergerissen.

Im übrigen Teil der Verpackung 10 sind die Verpackungsstreifen 12,13 weiterhin über die punktierten Streifenbereiche 14 zusammengehalten. In Pfeilrichtung 18 kann der Katheter 11 durch die Öffnung 17 hindurchgeschoben werden. Bevor der Katheter 11 durch die Öffnung 17 hindurchgeschoben wird, wird die Öffnung 17 mit der Gleithilfe 23 gefüllt. Dazu wird die Ampulle 20 in Pfeilrichtung 24 an die Öffnung 17 geführt, und über Fingerdruck wird die Gleithilfe 23 in die Öffnung 17 eingepreßt.

Alternativ zum Einpressen der Gleithilfe 23 von außen in die Öffnung 17 kann innerhalb unmittelbar hinter der Öffnung 17 eine Kapsel 25 angeordnet sein, die entweder aufgrund des Drucks der Spitze des Katheters 11 bzw. auf mechanischen Druck von außen aufsprengbar ist. Ist die Kapsel 25 gesprengt, fließt das Gleitmittel unmittelbar in die Öffnung 17 bzw. ist innerhalb der Verpackung 10 hinter der Öffnung 17 aufgestaut. Wird der Katheter 11 durch die Öffnung hindurchgeschoben, so wird bei diesem Vorgang die Außenoberfläche des Katheters 11 mit Gleitmittel benetzt.

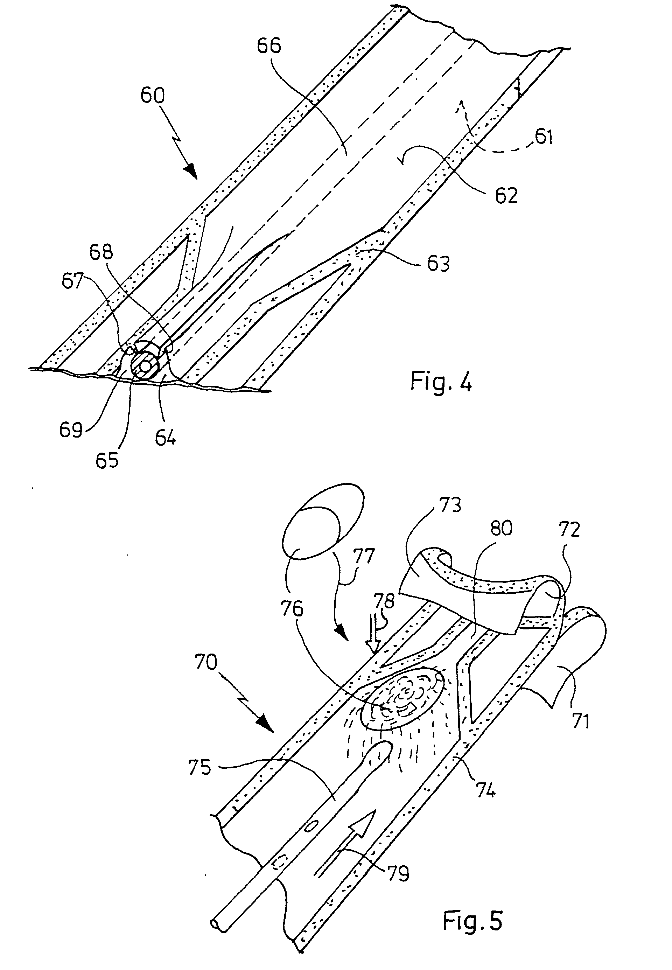

**Fig. 4** zeigt eine erfindungsgemäße Verpackung 60, die aus einem ersten Verpackungsstreifen 61 und einem zweiten Verpackungsstreifen 62 gebildet ist. Der Verpackungsstreifen 62 ist aus einem durchsichtigen Material gefertigt. Die Verpakkungsstreifen 61,62 sind in den punktierten Streifenbereichen 63 zusammengehalten. In der Fig. ist nur ein Teil der Verpackung 60 gezeigt. Sowohl das vordere wie das hintere Ende der Verpackung 60 sind abgeschnitten gezeigt. Durch eine Öffnung 64, die zwischen den Verpackungsstreifen 61,62 tunnelartig definiert ist und einen Kanal bildet, kann ein Katheter 65, von dem ein Teil eines Schaftabschnittes im Querschnitt gezeigt ist, in Pfeilrichtung 66 hindurchgeschoben werden. Innerhalb der tunnelförmigen Öffnung 64 sind Vorsprünge 67,68 ausgebildet, die in das Lumen der Öffnung 64 hineinragen. Zwischen den Vorsprüngen 67,68 bilden sich Freiräume, in denen Gleithilfe bzw. Gleitmittel speicherbar ist. Aus diesen Freiräumen kann das Gleitmittel an die Außenoberfläche des Katheters 65 dann abgegeben werden, wenn er durch die Öffnung 64 hindurchgeschoben wird. Bevor der Katheter 65 durch die Öffnung 64 hindurchgeschoben wird, wird die Öffnung 64 mit Gleithilfe 69 gefüllt.

**Fig. 5** zeigt einen weiteren Verpackungsendabschnitt einer Verpackung 70, die aus einem ersten Verpackungsstreifen 71 und einem zweiten Verpackungsstreifen 72 gebildet ist. Der erste Verpackungsstreifen 71 ist wiederum durchsichtig ausgebildet. Eine Öffnungshilfe 73 ist so weit geöffnet, daß Teile der punktierten Streifenbereiche 74 aufgerissen sind. Innerhalb der Verpackung 70 ist ein Katheter 75 angeordnet. Weiterhin ist innerhalb der Verpackung zwischen den Verpakkungsstreifen 71,72 eine Gleithilfekapsel 76 lagestabil gehalten, die mit dem Gleitmittel bzw. der Gleithilfe gefüllt ist. Die Gleithilfekapsel 76 ist der Übersichtlichkeit halber auch außerhalb der Verpackung nochmals dargestellt und die Pfeilrichtung 77 gibt an, an welcher Stelle sie innerhalb der Verpackung 17 lagestabil gehalten ist. Über mechanischen Druck in Pfeilrichtung 78 (Fingerdruck) kann die Gleithilfekapsel 76 gesprengt werden, und die in der Gleithilfekapsel 76 gespeicherte Gleithilfe fließt dann um den Katheter 75, wenn er in Pfeilrichtung 79 bewegt wird. Das Gleitmittel bzw. die Gleithilfe ist vor einer Öffnung 80 gestaut, in die die Gleithilfe dann mit hineingezogen wird, wenn der Katheter 75 durch die Öffnung 80 hindurchgeführt wird.

Eine erfindungsgemäße Verpackung 10 für die sterile Verpakkung eines medizinischen Instruments 11 weist eine Öffnung 17 zur Herausführung des medizinischen Instruments 11 aus der Verpackung 10 auf. Die Öffnung 17 ist in der Verpakkung 10 so ausgebildet, daß die Öffnung ein Benetzungsmittel im Bereich der Öffnung 17 speichern kann. Wird das medizinische Instrument 11 in Pfeilrichtung 18 durch die Öffnung hindurchgeführt, so wird die Außenoberfläche des medizinischen Instruments 11 benetzt. Bei der Verpackung 10 handelt es sich um eine an sich bekannte Verpackung, der eine weitere Funktion zugeordnet wird. Diese weitere Funktion kann die Verpackung übernehmen, ohne daß zusätzliche Teile der Verpackung 10 hinzuzufügen sind. Die Öffnung 17 ist Bestandteil der Verpackung 10 und aus der Verpackung 10 selbst geformt.

## Patentansprüche

1. Verpackung eines mit einem Benetzungsmittel behandelbaren medizinischen Instruments (11;65;75), insbesondere eines Katheters zum Einführen in die Urethra, wobei das medizinische Instrument (11;65;75) mindestens von einer Verpackung (10;60;70) steril umhüllt ist, und innerhalb der Verpackung (10;60;70) eine tunnelartige, derart gestaltete Öffnung (17;64;80) ausgebildet ist, daß das medizinische Instrument (11;65;75) seitlich geführt über das Lumen der Öffnung (17;64;80) herausschiebbar ist, und wobei die Verpackung (10;60;70) mindestens an einer der beiden Querseiten Öffnungshilfen (15;16;73) aufweist, **dadurch gekennzeichnet,** daß die Öffnungshilfen (15;16;73) zum Freilegen der tunnelartigen Öffnung (17;64;80) vorgesehen sind, daß das Benetzungsmittel im freigelegten Zustand der tunnelartigen Öffnung (17;64;80) unmittelbar in die nicht vom Katheter (11;65;75) besetzte tunnelartige Öffnung (17;64;80) einbringbar ist, und daß die tunnelartige Öffnung (17;64;80) so ausgebildet ist, daß nach Einbringen des Benetzungsmittels dieses im Lumen der tunnelartigen Öffnung (17;64;80) so gespeichert wird, dass das Benetzungsmittel an die Aussoberfläche eines durch die Öffnung geführten medizinischen Instruments abgebbar ist.

2. Verpackung eines medizinischen Instruments nach Anspruch 1 **dadurch gekennzeichnet,** daß die Verpackung (10;60;70) in der Größe dem medizinischen Instrument (11;65;75) angepaßt ist und daß die Verpackung (10;60;70) mindestens auf einer Seite weitgehend durchsichtig ausgebildet ist.

3. Verpackung eines medizinischen Instruments nach einem der Ansprüche 1 oder 2, **dadurch gekennzeichnet,** daß die Verpackung (10;60;70) aus einem ersten Verpackungsstreifen (12;61;71) und einem zweiten Verpackungsstreifen (13;62;72) gebildet ist, die aufeinanderliegend im Randbereich (14;63;64) allseitig lösbar miteinander verbunden sind.

4. Verpackung eines medizinischen Instruments nach Anspruch 3, **dadurch gekennzeichnet,** daß die Verpackungsstreifen (12,13;61,62;71,72) derart im Bereich der Öffnung (17;64;80) zusammengefügt sind, daß die seitliche Führung des medizinischen Instruments (11;65;75) gewährt ist.

5. Verpackung eines medizinischen Instruments nach einem der Ansprüche 1 bis 4, **dadurch gekennzeichnet**, daß das Benetzungsmittel eine Gleithilfe (23;69) oder ein einen medizinischen Wirkstoff beinhaltendes Gleitmittel ist, die oder das in einer Ampulle (20) oder einer Kapsel (25) gespeichert ist.

6. Verpackung eines medizinischen Instruments nach einem der vorhergehenden Ansprüche, **dadurch gekennzeichnet,** daß unmittelbar hinter der Öffnung (80) innerhalb der Verpackung (70) eine unter Druck zerstörbare Gleithilfekapsel (76) lagestabil angeordnet ist.

7. Verpackung eines medizinischen Instruments nach einem der Ansprüche 1 bis 6, **dadurch gekennzeichnet,** daß an der Verpackung (60) im Bereich der Öffnung (64) über eine axiale Erstreckung Vorsprünge (67,68) in das Lumen der Öffnung (64) ragen.

## Claims

1. A packing of a medical instrument (11;65;75) which can be treated with a wetting agent, in particular, a catheter for insertion into the urethra, wherein the medical instrument (11;65;75) is covered in a sterile fashion by at least one packing (10;60;70), and a tunnel-like opening (17;64;80) is formed within the packing (10;60;70), designed such that the medical instrument (11;65;75) is laterally guided to be pushed out via the lumen of the opening (17;64;80) and wherein the packing (10;60;70) comprises opening aids (15;16;73) on at least one of the two transverse sides, characterized in that the opening aids (15;16;73) are provided for exposing the tunnel-shaped opening (17;64;80), that the wetting agent can be introduced, in the exposed state of the tunnel-like opening (17;64;80), directly into the tunnel-like opening (17;64;80) which is not occupied by the catheter (11;65;75), and that the tunnel-like opening (17;64;80) is designed such that after insertion of the wetting agent, same is stored in the lumen of the tunnel-like opening (17;64;80) such that the wetting agent can be transferred to the outer surface of a medical instrument guided through the opening.

2. The packing of a medical instrument according to claim 1, characterized in that the size of the packing (10;60;70) is adapted to the medical instrument (11;65;75) and that the packing (10;60;70) is largely transparent on at least one side.

3. The packing of a medical instrument according to any one of the claims 1 or 2, characterized in that the packing (10;60;70) is formed of a first packing strip (12;61;71) and a second packing strip (13;62;72) which, disposed on top of one another, are detachably connected on all sides in the edge area (14;63;64).

4. The packing of a medical instrument according to claim 3, characterized in that the packing strips (12,13;61,62;71,72) are connected in the area of the opening (17;64;80) in such a fashion, that lateral guidance of the medical instrument (11;65;75) is guaranteed.

5. The packing of a medical instrument according to any one of the claims 1 through 4, characterized in that the wetting agent is a sliding aid (23;69) or a sliding agent containing a medically active substance which is stored in an ampoule (20) or capsule (25).

6. The packing of a medical instrument according to any one of the preceding claims, characterized in that a sliding aid capsule (76) is disposed in a positionally stable fashion directly behind the opening (80) within the packing (70) which can be destroyed by pressure.

7. The packing of a medical instrument according to any one of the claims 1 through 6, characterized in that projections (67,68) project, via an axial extension, into the lumen of the opening (64) in the region of the opening (64) of the packing (60).

## Revendications

1. Emballage pour instrument médical (11; 65; 75) pouvant être traité avec un agent mouillant, notamment un cathéter destiné à être introduit dans l'urètre, et dans lequel l'instrument médical (11; 65; 75) est enveloppé d'une manière stérile au moins par un emballage (10; 60; 70), et à l'intérieur de l'emballage (10; 60; 70) est formée une ouverture en forme de tunnel (17; 64; 80) agencée de telle sorte qu'on peut faire ressortir l'instrument médical (11; 65; 75), d'une manière guidée latéralement, par la lumière de l'ouverture (17, 62; 80), et dans lequel l'emballage (10; 60; 70) comporte des systèmes auxiliaires d'ouverture (15; 16; 73) au moins sur l'un des deux côtés transversaux, caractérisé en ce que les systèmes auxiliaires d'ouverture (15; 16; 73) sont prévus pour libérer l'ouverture en forme de tunnel (17; 64; 80), que, lorsque l'ouverture en forme de tunnel (17; 64; 80) est dans l'état libéré, l'agent mouillant peut être introduit directement dans l'ouverture en forme de tunnel (17; 64; 80) non occupée par le cathéter (11; 65; 75) et que l'ouverture en forme de tunnel (17; 64; 80) est agencée de telle sorte qu'après insertion de l'agent mouillant, ce dernier est stocké dans la lumière de l'ouverture en forme de tunnel (17; 64; 80) de sorte que l'agent mouillant peut être délivré sur la surface extérieure d'un instrument médical guidé dans l'ouverture.

2. Emballage pour instrument médical selon la revendication 1, caractérisé en ce que l'emballage (10; 60; 70) a une taille adaptée à celle de l'instrument médical (11; 65; 75) et que l'emballage (10; 60; 70) est agencé de manière à être dans une large mesure transparent au moins sur un côté.

3. Emballage pour instrument médical selon la revendication 1 ou 2, caractérisé en ce que l'emballage (10; 60; 70) est constitué par une première bande d'emballage (12; 61; 71) et par une seconde bande d'emballage (13; 62; 72), qui sont reliées entre elles de façon amovible, de tous côtés, en étant superposées l'une sur l'autre dans la zone de bord (14; 63; 64).

4. Emballage pour instrument médical selon la revendication 3, caractérisé en ce que les bandes d'emballage (12, 13; 61, 62; 71, 72) sont réunies dans la zone de l'ouverture (17; 64; 80) de telle sorte que le guidage naturel de l'instrument médical (11; 65; 75) est garanti.

5. Emballage pour instrument médical selon la revendication 1 à 4, caractérisé en ce que l'agent mouillant est un agent facilitant le glissement (3; 69) ou un agent facilitant le glissement contenant une substance active médical et qui est stocké dans une ampoule (20) ou dans une capsule (25).

6. Emballage pour instrument médical selon les revendications précédentes, caractérisé en ce qu'une capsule (76), qui contient la substance favorisant le glissement et peut être détruite sous l'action d'une pression, est montée dans une position stable directement en arrière de l'ouverture (80), à l'intérieur de l'emballage (70).

7. Emballage pour instrument médical selon l'une des revendications 1 à 6, caractérisé en ce que sur l'emballage (60), dans la zone de l'ouverture (75), des parties saillantes (67, 68) pénètrent sur une étendue axiale, dans la lumière de l'ouverture (64).
